# EUROPÄISCHE PATENTSCHRIFT

(11) **EP 0 848 600 B1**
(45) Veröffentlichungstag und Bekanntmachung des Hinweises auf die Patenterteilung: **09.05.2001**
(21) Anmeldenummer: 95931996.3
(22) Anmeldetag: 06.09.1995
(51) Int. Cl.: A61B 17/80, A61B 17/86

(54) **KNOCHENPLATTE**
BONE PLATE
PLAQUE POUR LIAISON OSSEUSE

(43) Veröffentlichungstag der Anmeldung: 24.06.1998
(73) Patentinhaber: SYNTHES AG Chur, 7002 Chur (CH)
(72) Erfinder: FRIGG, Robert, CH-7270 Davos Platz (CH); SCHAVAN, Robert, CH-7260 Davos Dorf (CH); HEHLI, Markus, CH-7276 Davos Frauenkirch (CH)
(74) Vertreter: Lusuardi, Werther Giovanni, Dr.
(86) Internationale Anmeldenummer: EP9503494
(87) Internationale Veröffentlichungsnummer: WO9709000

(56) Entgegenhaltungen:
- EP-A- 0 486 762
- EP-A- 0 530 585
- WO-A-90/07304
- DE-A- 4 341 980
- DE-A- 4 343 117
- FR-A- 2 667 913
- FR-A- 2 706 763
- US-A- 3 596 656

## Beschreibung

Die Erfindung bezieht sich auf eine Knochenplatte gemäss dem Oberbegriff des Patentanspruchs 1.

Eine solche Knochenplatte ist aus der EP-A 0 530 585 bekannt. Die dauerhafte Fixation von Knochenplatten mit konischen Plattenlöchern mittels Knochenschrauben mit strukturiertem konischem Kopf hängt von der erzielbaren Formschlüssigkeit der Verbindung zwischen Schraubenkopf und Plattenloch ab, welche vorallem bei kleineren Dimensionen problematisch ist.
Im weiteren ist ein Fixationssystem für Knochen aus der DE-A 43 43 117 bekannt, welches aus einer metallischen Knochenplatte mit gewindetragenden Plattenbohrungen und Knochenschrauben mit einem sphärischen Kopf besteht, wobei die gewindetragende sphärische Sitzfläche der Knochenschraube härter ist als die Knochenplatte.

Hier will die Erfindung Abhilfe schaffen. Der Erfindung liegt die Aufgabe zugrunde, eine Knochenplatte der genannten Gattung zu schaffen, welche eine optimale Formschlüssigkeit mit den zu ihrer Fixation bestimmten Knochenschrauben garantiert.

Die Erfindung löst die gestellte Aufgabe mit einer Knochenplatte, welche die Merkmale des Anspruchs 1 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Ausgestaltung der strukturierten Mantelfläche des zur Fixation der Knochenplatte bestimmten Schraubenkopfs eine hervorragende Formschlüssigkeit mit der Knochenplatte erzielt werden kann.

Die Erfindung und Weiterbildungen der Erfindung werden im folgenden anhand der teilweise schematischen Darstellungen mehrerer Ausführungsbeispiele noch näher erläutert.
Es zeigen :
Fig. 1 einen Längsschnitt durch die erfindungsgemässe Knochenplatte;
Fig. 2 eine perspektivische Darstellung einer Knochenschraube zur Fixation der Knochenplatte nach Fig. 1; und
Fig. 3 eine perspektivische Teilansicht der Oberseite der Knochenplatte nach Fig. 1.

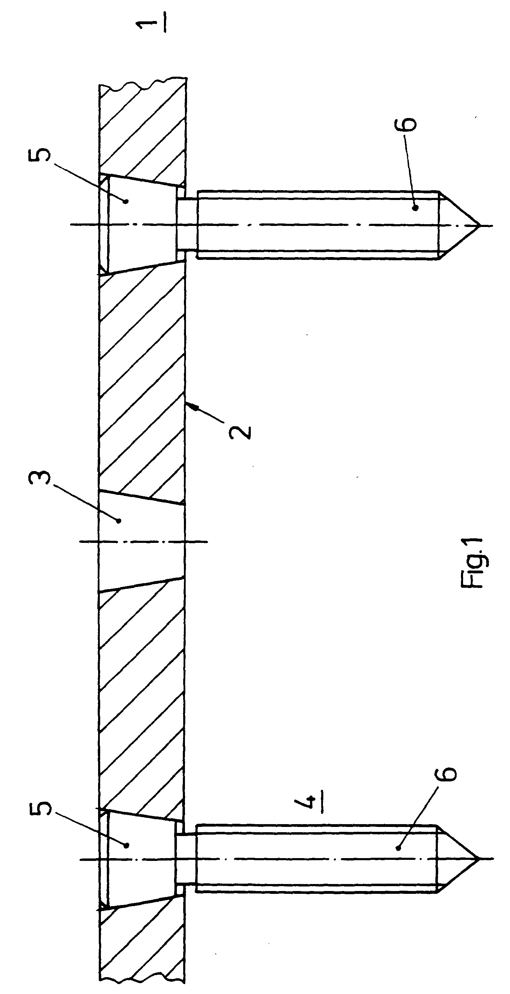

Die in den Fig. 1 - 3 dargestellte Knochenplatte 1 besitzt mehrere sich zur Knochenkontaktfläche 2 hin verjüngende konische Plattenlöcher 3, in welche Knochenschrauben 4 einführbar sind. Die Knochenschrauben 4 sind mit einem zur Einführung in die konischen Plattenlöcher 3 bestimmten, darauf abgestimmten, konischen Kopfteil 5 mit strukturierter Mantelfläche 7 und einem zur Verankerung im Knochen bestimmten Gewindeteil 6 versehen.
Die strukturierte Mantelfläche 7 des Kopfteils 5 der Knochenschrauben 4 weist eine höhere Härte als das Material der Knochenplatte 1 im Bereich ihrer Plattenlöcher 3 auf. Dies hat zur Folge, dass auch bei einer nicht völlig koaxialen Einführung der Knochenschrauben 4 in das Plattenloch 3 eine optimale Formschlüssigkeit erzielt wird, da die härtere, strukturierte Mantelfläche 7 in das weichere Material des Plattenlochs 3 eindringen kann.

Die Härte der strukturierten Mantelfläche 7 wird vorteilhafterweise im Bereich von 230 - 450 HV (Vickers Härtegrade), vorzugsweise im Bereich von 250 - 350 HV, gewählt. Als Material für die Knochenplatte 1 eignet sich ein Metall der Härte 100 - 220, vorzugsweise von 120 - 200 HV. Vorteilhafterweise beträgt die Härte der strukturierten Mantelfläche 7 mindestens das Doppelte der Härte der Knochenplatte 1 im Bereich ihrer Plattenlöcher 3.

Die strukturierte Mantelfläche 7 weist vorzugsweise eine quer zur Längsachse der Knochenschraube 4 verlaufende Strukturierung, z.B. in Form von peripher umlaufenden Rippen, auf. Dies führt zu einer weiteren Verbesserung der Formschlüssigkeit.

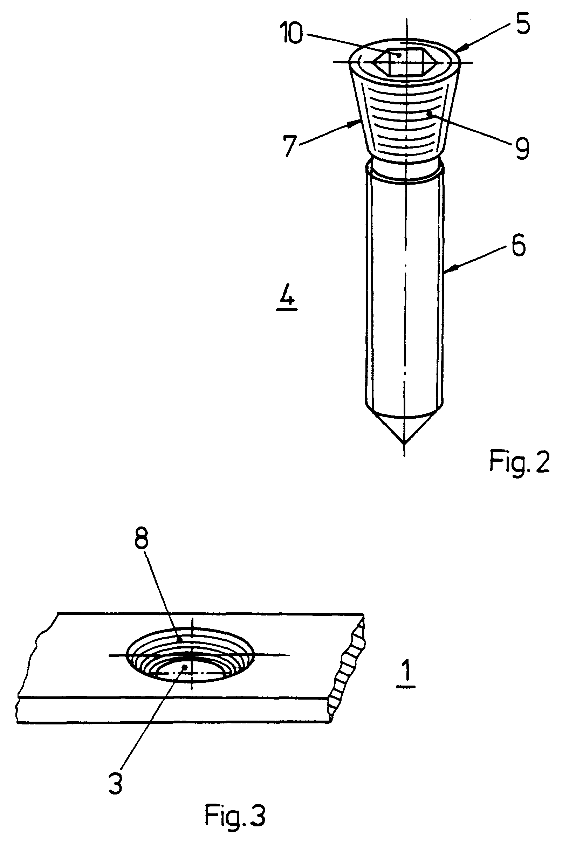

Bei einer bevorzugten Ausführungsform der Erfindung, wie sie in Fig. 2 dargestellt ist, ist die Mantelfläche 7 des Kopfteils 5 der Knochenschrauben 4 mit einem Gewinde 9 oder spiralförmigen Strukturen versehen.
Wie in Fig. 3 dargestellt können die Plattenlöcher 3 ebenfalls mit einem Gewinde 8 versehen sein. Vorteilhafterweise sind die beiden Gewinde 8,9 aufeinander abgestimmt. Bei einer solchen Kombination ist es vorteilhaft, wenn das Gewinde 9 des Kopfteils 5 der Knochenschrauben 4 bezüglich des Gewindes 8 der Plattenlöcher 3 zwei oder mehrgängig ausgebildet ist.
Der Kopfteil 5 der Knochenschraube 4 weist an seinem oberen freien Ende eine sechskantige Vertiefung 10 auf, zur Aufnahme eines Sechskant-Schraubenziehers.

Statt den Kopfteil 5 der Knochenschraube 4 insgesamt aus einem härteren Material als die Knochenplatte 1 zu realisieren, kann dieser auch nur mit einer Beschichtung versehen sein, welche eine höhere Härte als die Knochenplatte 1, im Bereich ihrer Plattenlöcher 3 aufweist. Diese Beschichtung kann eine Oberflächenhärte von 500 - 10'000, vorzugsweise von 1000 - 5'000 HV aufweisen.

Vorzugsweise besteht die Knochenplatte 1 aus einem Kunststoff und die Knochenschrauben 4 aus Metall oder Keramik. Als Kunststoffe für die Knochenplatte 1 eignen sich insbesondere verstärkte oder unverstärkte Kunststoffe des Typs:
- Polyacryletherketon (PEAK) oder Polyetheretherketon (PEEK) mit einer Bruchdehnung von 40 - 70 % und einem Elastizitätsmodul von 3000 - 6000 N/mm²;
- Polysulfon mit einer Bruchdehnung von 80 - 120 % und einem Elastizitätsmodul von 2000 - 3500 N/mm²;
- Liquid-Cristal-Polymer (LCP) mit einer Bruchdehnung von 1,5 - 2,5 % und einem Elastizitätsmodul von 5000 - 20'000 N/mm²;
- Polyoxymethylen (POM) mit einer Bruchdehnung von 10 - 50 % und einem Elastizitätsmodul von 2000 - 3'500 N/mm²; und
- Polyphenylensulfid (PPS) mit einer Bruchdehnung von 0,2 - 1,0 % und einem Elastizitätsmodul von 12000 - 20'000 N/mm².

Die Verstärkung des Kunststoffs kann durch Einbettung von Metall-, Kunststoff- oder Kohlenstoff-Fasern in die Kunststoffmatrix erfolgen.

## Patentansprüche

1. Knochenplatte (1) mit mehreren sich zur Knochenkontaktfläche (2) hin verjüngenden konischen Plattenlöchern (3) und mindestens zwei zur Verankerung der Knochenplatte (1) bestimmten Knochenschrauben (4) mit einem zur Einführung in die konischen Plattenlöcher (3) bestimmten, darauf abgestimmten, konischen Kopfteil (5) mit strukturierter Mantelfläche (7) und einem zur Verankerung im Knochen bestimmten Gewindeteil (6),
**dadurch gekennzeichnet, dass**
A) die strukturierte Mantelfläche (7) eine höhere Härte als das Material der Knochenplatte (1) im Bereich ihrer Plattenlöcher (3) aufweist; und
B) die Härte der strukturierten Mantelfläche (7) mindestens doppelt so gross ist, wie die Härte der Knochenplatte (1) im Bereich ihrer Plattenlöcher (3).

2. Knochenplatte (1) nach Anspruch 1, dadurch gekennzeichnet, dass die Härte der strukturierten Mantelfläche (7) 230 - 450 HV, vorzugsweise 250 - 350 HV beträgt.

3. Knochenplatte (1) nach Anspruch 1 oder 2, dadurch gekennzeichnet, dass sie aus einem Metall der Härte 100 - 220 HV, vorzugsweise von 120 - 200 HV besteht.

4. Knochenplatte (1) nach einem der Ansprüche 1 - 3, dadurch gekennzeichnet, dass die Mantelfläche (7) des Kopfteils (5) der Knochenschrauben (4) mit einem Gewinde (9) oder spiralförmigen Strukturen versehen ist.

5. Knochenplatte (1) nach einem der Ansprüche 1 bis 4, dadurch gekennzeichnet, dass die Plattenlöcher (3) mit einem Gewinde (8) versehen sind.

6. Knochenplatte (1) nach einem der Ansprüche 1 bis 5, dadurch gekennzeichnet, dass der Kopfteil (5) der Knochenschrauben (4) mit einer Beschichtung versehen ist, welche eine höhere Härte als die Knochenplatte (1) im Bereich ihrer Plattenlöcher (3) aufweist.

7. Knochenplatte (1) nach Anspruch 6, dadurch gekennzeichnet, dass die Beschichtung eine Oberflächenhärte von 500 - 10'000 HV aufweist.

8. Knochenplatte (1) nach Anspruch 7, dadurch gekennzeichnet, dass die Beschichtung eine Oberflächenhärte von 1000 - 5'000 HV aufweist.

9. Knochenplatte (1) nach einem der Ansprüche 5 bis 8, dadurch gekennzeichnet, dass die strukturierte Mantelfläche (7) des Kopfteils (5) der Knochenschrauben (4) und die Plattenlöcher (3) aufeinander abgestimmte Gewinde (8,9) aufweisen.

10. Knochenplatte (1) nach einem der Ansprüche 5 bis 8, dadurch gekennzeichnet, dass das Gewinde (9) des Kopfteils (5) der Knochenschrauben (4) bezüglich des Gewindes (8) der Plattenlöcher (3) zwei oder mehrgängig ausgebildet ist.

11. Knochenplatte (1) nach einem der Ansprüche 1 bis 10, dadurch gekennzeichnet, dass die strukturierte Mantelfläche (7) eine quer zur Längsachse der Knochenschraube (4) verlaufende Strukturierung, vorzugsweise von peripher umlaufenden Rippen, aufweist.

12. Knochenplatte (1) nach einem der Ansprüche 1 bis 11, dadurch gekennzeichnet, dass die Knochenplatte (1) aus einem Kunststoff und die Knochenschrauben (4) aus Metall oder Keramik gefertigt sind.

13. Knochenplatte (1) nach einem der Ansprüche 1 bis 12, dadurch gekennzeichnet, dass die Knochenplatte (1) aus Polyacryletherketon (PEAK) oder Polyetheretherketon (PEEK) mit einer Bruchdehnung von 40 - 70 % und einem Elastizitätsmodul von 3000 - 6000 N/mm² gefertigt ist.

14. Knochenplatte (1) nach einem der Ansprüche 1 bis 12, dadurch gekennzeichnet, dass die Knochenplatte (1) aus Polysulfon mit einer Bruchdehnung von 80 - 120 % und einem Elastizitätsmodul von 2000 - 3500 N/mm² gefertigt ist.

15. Knochenplatte (1) nach einem der Ansprüche 1 bis 12, dadurch gekennzeichnet, dass die Knochenplatte (1) aus Liquid-Cristal-Polymer (LCP) mit einer Bruchdehnung von 1,5 - 2,5 % und einem Elastizitätsmodul von 5000 - 20'000 N/mm² gefertigt ist.

16. Knochenplatte (1) nach einem der Ansprüche 1 bis 12, dadurch gekennzeichnet, dass die Knochenplatte (1) aus Polyoxymethylen (POM) mit einer Bruchdehnung von 10 - 50 % und einem Elastizitätsmodul von 2000 - 3'500 N/mm² gefertigt ist.

17. Knochenplatte (1) nach einem der Ansprüche 1 bis 12, dadurch gekennzeichnet, dass die Knochenplatte (1) aus Polyphenylensulfid (PPS) mit einer Bruchdehnung von 0,2 - 1,0 % und einem Elastizitätsmodul von 12000 - 20'000 N/mm² gefertigt ist.

18. Knochenplatte (1) nach einem der Ansprüche 1 bis 17, dadurch gekennzeichnet, dass die Knochenplatte (1) aus einem mit Metall-, Kunststoff- oder Kohlenstoff-Fasern verstärkten Kunststoff gefertigt ist.

## Claims

1. A bone plate (1) with a plurality of concial plate holes (3) tapering toward a bone contact surface (2) and with at least two bone screws (4) for anchoring the bone plate (1) to a bone, each of said screws having a conical head (5) with a textured lateral outside surface (7), said head being shaped to match the plate holes (3), the bone screws (4) further comprising a threaded shank (6) for anchoring into the bone,
characterized in that
A) the structured lateral outside surface (7) has greater hardness than that material of the bone plate (1) which is located in the vicinity of its plate holes (3); and
B) the hardness of the structured lateral outside surface (7) is at least twice as high than the hardness of the bone plate (1) in the vicinity of its plate holes (3).

2. Bone plate according to claim 1, characterized in that the hardness of the structured lateral outside surface (7) is 230 to 450 HV and preferably 250 to 350 HV.

3. Bone plate according to claim 1 or 2, characterized in that it consists of a metal with hardness of 100 to 220 HV, preferably 120 to 200 HV.

4. Bone plate according to one of the claims 1 to 3, characterized in that the lateral outside surface (7) of the head (5) of the bone screws (4) is fitted with a thread (9) or with spiral structures.

5. Bone plate according to one of the claims 1 to 4, characterized in that the plate holes (3) are fitted with a thread (8).

6. Bone plate according to one of the claims 1 to 5, characterized in that the head (5) of the bone screws (4) is fitted with a coating of higher hardness than the bone plate (1) in the vicinity of its plate holes (3).

7. Bone plate according to claim 6, characterized in that the coating evinces a surface hardness of 500 - 10'000 HV.

8. Bone plate according to claim 7, characterized in that the coating evinces a surface hardness of 1000 - 5000 HV.

9. Bone plate according to one of the claims 5 to 8, characterized in that the structured lateral outside surface (7) of the head (5) of the bone screws (4) and the plate holes (3) evince mutually matching threads (8, 9).

10. Bone plate according to one of the claims 5 to 8, characterized in that the thread (9) of the head (5) of the bone screws (4) comprises one or more turns relative to the thread (8) of the plate holes (3).

11. Bone plate according to one of the claims 1 to 10, characterized in that the structural lateral outside surface (7) comprises a structure, preferably circumferential ribs, running transversely to the longitudinal axis of the bone screw (4).

12. Bone plate according to one of the claims 1 to 11, characterized in that the bone plate (1) is made of a plastic and the bone screws (4) are made of metal or ceramics.

13. Bone plate according to one of the claims 1 to 12, characterized in that the bone plate (1) is made of polyacrylic ether ketone (PEAK) or of polyether ether ketone (PEEK) with a rupture elongation of 40 to 70 % and with a Young's modulus of 3'000 to 6'000 N/mm².

14. Bone plate according to one of the claims 1 to 12, characterized in that the bone plate (1) is made of a polysulfone with a rupture elongation of 80 to 120 % and with a Young's modulus of 2'000 to 3'500 N/mm².

15. Bone plate according to one of the claims 1 to 12, characterized in that the bone plate (1) is made of a Liquid Crystal Polymer (LCP) with a rupture elongation of 1,5 to 2,5 % and with a Young's modulus of 5'000 to 20'000 N/mm².

16. Bone plate according to one of the claims 1 to 12, characterized in that the bone plate (1) is made of a polyoxymethylene (POM) with a rupture elongation of 10 to 50 % and with a Young's modulus of 2'000 to 3'500 N/mm².

17. Bone plate according to one of the claims 1 to 12, characterized in that the bone plate (1) is made of a polyethylene sulfide (PPS) with a rupture elongation of 0,2 to 1,0 % and with a Young's modulus of 12'000 to 20'000 N/mm².

18. Bone plate according to one of the claims 1 to 17, characterized in that the bone plate (1) is made of a plastic reinforced with metal fibers, plastic fibers or carbon fibers.

## Revendications

1. Plaque (1) pour liaison osseuse présentant plusieurs trous coniques de plaque (3) qui se rétrécissent en direction de la surface (2) de contact avec l'os, et au moins deux vis pour os (4) destinées à l'ancrage de la plaque pour liaison osseuse (1) et qui présentent une partie conique de tête (5) à surface d'enveloppe (7) structurée, destinées à l'insertion dans les trous coniques de plaque (3), et une partie filetée (6) destinée à l'ancrage dans l'os,
caractérisée en ce que
A) la surface d'enveloppe (7) structurée présente une dureté supérieure à celle du matériau de la plaque pour os (1) dans la région de ses trous de plaque (3) ; et
B) la dureté de la surface d'enveloppe (7) structurée vaut au moins le double de la dureté de la plaque pour os (1) dans la région de ses trous de plaque (3).

2. Plaque (1) pour liaison osseuse selon la revendication 1, caractérisée en ce que la dureté de la surface d'enveloppe (7) structurée est de 230 à 450 HV, de préférence de 250 à 350 HV.

3. Plaque (1) pour liaison osseuse selon la revendication 1 ou 2, caractérisée en ce qu'elle est constituée d'un métal dont la dureté est de 100 à 220 HV, de préférence de 120 à 200 HV.

4. Plaque (1) pour liaison osseuse selon l'une des revendications 1 à 3, caractérisée en ce que la surface d'enveloppe (7) de la partie de tête (5) de la vis pour os (4) est dotée d'un filet (9) ou d'une structuration en spirale.

5. Plaque (1) pour liaison osseuse selon l'une des revendications 1 à 4, caractérisée en ce que les trous de plaque (3) sont dotés d'un filet (8).

6. Plaque (1) pour liaison osseuse selon l'une des revendications 1 à 5, caractérisée en ce que la partie de tête (5) des vis pour os (4) est dotée d'un revêtement qui présente une dureté plus élevée que celle de la plaque (1) pour liaison osseuse dans la région de ses trous de plaque (3).

7. Plaque (1) pour liaison osseuse selon la revendication 6, caractérisée en ce que le revêtement présente une dureté superficielle de 500 à 10 000 HV.

8. Plaque (1) pour liaison osseuse selon la revendication 7,
caractérisée en ce que le revêtement présente une dureté superficielle de 1 000 à 5 000 HV.

9. Plaque (1) pour liaison osseuse selon l'une des revendications 5 à 8, caractérisée en ce que la surface d'enveloppe (7) structurée de la partie de tête (5) des vis pour os (4) et les trous de plaque (3) présentent des filets (8, 9) complémentaires l'un de l'autre.

10. Plaque (1) pour liaison osseuse selon l'une des revendications 5 à 8, caractérisée en ce que le filet (9) de la partie de tête (5) des vis pour os (4) est configurée double ou multiple par rapport au filet (8) des trous de plaque (3).

11. Plaque (1) pour liaison osseuse selon l'une des revendications 1 à 10, caractérisée en ce que la surface d'enveloppe (7) structurée présente une structuration qui s'étend transversalement par rapport à l'axe longitudinal de la vis pour os (4), de préférence des nervures périphériques.

12. Plaque (1) pour liaison osseuse selon l'une des revendications 1 à 11, caractérisée en ce que la plaque (1) pour liaison osseuse est réalisée en une matière plastique et les vis pour os (4) en métal ou en céramique.

13. Plaque (1) pour liaison osseuse selon l'une des revendications 1 à 12, caractérisée en ce que la plaque pour liaison osseuse (1) est réalisée en polyacryléthercétone (PEAC) ou en polyétheréthercétone (PEEC) dont l'allongement à la rupture est de 40 à 70 % et le module d'élasticité de 3 000 à 6 000 N/mm².

14. Plaque (1) pour liaison osseuse selon l'une des revendications 1 à 12, caractérisée en ce que la plaque pour liaison osseuse (1) est réalisée en polysulfone dont l'allongement à la rupture est de 80 à 120 % et le module d'élasticité de 2 000 à 3 500 N/mm².

15. Plaque (1) pour liaison osseuse selon l'une des revendications 1 à 12, caractérisée en ce que la plaque pour liaison osseuse (1) est réalisée en polymère à cristaux liquide (PCL) dont l'allongement à la rupture est de 1,5 à 2,5 % et le module d'élasticité de 5 000 à 20 000 N/mm².

16. Plaque (1) pour liaison osseuse selon l'une des revendications 1 à 12, caractérisée en ce que la plaque pour liaison osseuse (1) est réalisée en polyoxyméthylène (POM) dont l'allongement à la rupture est de 10 à 50 % et le module d'élasticité de 2 000 à 3 500 N/mm².

17. Plaque (1) pour liaison osseuse selon l'une des revendications 1 à 12, caractérisée en ce que la plaque pour liaison osseuse (1) est réalisée en poly(sulfure de phénylène) (PSP) dont l'allongement à la rupture est de 0,2 à 1,0 % et le module d'élasticité de 12 000 à 20 000 N/mm².

18. Plaque (1) pour liaison osseuse selon l'une des revendications 1 à 17, caractérisée en ce que la plaque pour liaison osseuse (1) est réalisée en une matière synthétique renforcée de fibres en métal, en matière synthétique ou en carbone.
